# EUROPEAN PATENT APPLICATION

(11) **EP 3 928 795 A1**
(43) Date of publication of application: **29.12.2021**
(21) Application number: 20182648.4
(22) Date of filing: 26.06.2020
(51) Int. Cl.: A61K 47/02, A61K 38/08

(54) **USE OF A BORON CLUSTER COMPOUND AS TRANSMEMBRANE CARRIER**

(71) Applicant: Jacobs University Bremen gGmbH, 28759 Bremen (DE); University of Santiago de Compostela, 15782 Santiago de Compostela (ES)
(72) Inventor: Nau, Werner, 28759 Bremen (DE); Montenegro, Javier, 15782 Santiago de Compostela (ES)
(74) Representative: Weidner Stern Jeschke

(57) **Abstract**

The invention relates to the use of a boron cluster compound as transmembrane carrier to transport a bioactive molecule across a membrane of a cell or vesicle.

## Description

The invention relates to the use of a boron cluster compound as transmembrane carrier to transport a bioactive molecule across a membrane.

For medical or biological agents to be effective, they must reach their biomolecular target, which is usually located inside the cell. The agent must thus penetrate the cell membrane, which forms a natural barrier against foreign substances. For penetration, either the active ingredients may be chemically modified, which often changes or weakens the agent's effect, or additives can be added, which serve as carriers (counterion activators) through the membrane. Such effective transporters are commercially applied in medicine and pharmacy, but they are also commonly used in cell biology and basic research. Membrane transport and intracellular delivery constitutes a major bottleneck for the discovery and application of new therapeutics and bioactive compounds in drug delivery and molecular biology.

As transporters, a frequently applied strategy is the use of organic cationic molecules with aromatic or aliphatic hydrophobic moieties, which enhance multivalent interactions with the cell membrane and thereby trigger the translocation of the cargo. However, these carriers and their cargo complexes usually enter cells by endocytosis, which hinders the intracellular targeting of the cargo. To remedy, elaborate strategies have been developed, such as the introduction of hydrophobic carbon tails, the coupling with membrane-lytic peptides, the development of pH-sensitive peptides (e.g. GALA) or the use of strongly amphiphilic peptides and polymers.

An intriguing alternative to by-pass the interference of endosomal entrapment is the use of anionic amphiphilic activators. These activators neutralize the charge of cationic carriers and increase their hydrophobicity, which switches their endosomal uptake to direct membrane translocation. However, the amphiphilic transporters currently used to transport cationic agents have several disadvantages: Many precipitate the agents when they are mixed in solution, so that the transporter has to be added first; this is not feasible for pharmaceuticals. Although many transporters promote the uptake of the agents, this often occurs by an endosomal transport mechanism that may not lead to the release of the agents in the cytosol, but instead transports the active ingredients out of the cell. Many carriers can only be applied to a small number of compounds. Due to the transporters' amphiphilicity (they contain a hydrophobic group), the transporters are furthermore only slightly water soluble. Even though many transporters are active in liposomes (which serve as membrane models), the results are often not transferable to cells. For positively charged and neutral compounds, for example to introduce antimicrobial peptides into cells, only a few transporters are available so far.

### Summary of the invention

It is therefore an objective of the present invention to provide a method which allows a transport of compounds inside of cells and/or vesicles but overcomes the described shortcomings, in particular avoid precipitation, allow or circumvent endosomal escape and allow a transport of various substance classes with one type of carrier to avoid the need for optimization.

This objective is met by a use of a boron cluster compound as transmembrane carrier to transport a bioactive molecule across a membrane of a cell or vesicle.

The essence of the invention is therefore that different molecules can be introduced into vesicles or cells by means of boron clusters. It could be shown that boron clusters can function as a new class of membrane transporters. Boron clusters are therefore suitable for transporting other molecules through the membrane of cells or vesicles.

Advantages of boron clusters over the amphiphilic transporters used in the state of the art are good water solubility, broad applicability ("broadband"), and they do not precipitate the agents when added to the solution. They can be chemically modified and are accessible on a large scale.

Antibiotics, biocides or drugs can thus be transported more readily into cells and their effect can thus be enhanced. In the same way, dyes can be transported into cells by means of boron clusters, which means that otherwise disadvantageous methods or reagents can be avoided. Boron clusters can be used to transport peptides, antibiotics, dyes, proteins and other molecules, especially positively charged molecules, into the interior of vesicles (liposomes) or into cells. The vesicles may be used as membrane models. Especially the transport of dyes and antibiotics is possible, boron clusters can thereby be used to bypass antibiotic resistance.

The further advantages of the boron clusters include, most importantly, their potential to facilitate or bypass their endosomal escape and their broad cargo scope; the latter ranges from the protein protamine to arginine- and even lysine-containing charged peptides, to neutral cyclopeptides, to low-molecular weight analytes and drugs such as selected antibiotics.

In contrast to the conventional cationic, amphiphilic, and encapsulating carriers, boron clusters are highly watersoluble and do neither encapsulate nor tend to form nanoscale aggregates with their corresponding cargo. This leads commonly to complexation and precipitation with the compounds used in the state of the art.

Their effectiveness in inducing endosomal release, their biocompatibility, the transferability of their activity from vesicles to cells, and their broad cargo scope, which includes the delivery of impermeable small and neutral molecules, peptides, and proteins, differentiates them from known carriers and amphiphilic counterion activators. Boron clusters act as transmembrane carriers and, at the same time, counterion activators. The transferability of the carrier activity from vesicles to live cells, the high water solubility of the clusters (and their salts), insensitivity to membrane potential changes, the full retention of biological activity upon inversion of the sequence of cargo-carrier addition, are added assets that amphiphilic carriers have been lacking.

Membranes of living cells can also be penetrated and the compounds used are not cytotoxic and can therefore be used for living cells. The transport observed in the vesicles, which is induced by the boron clusters, is therefore also transferable to living cells respectively is also observed in them.

A "transmembrane carrier" is understood as molecule that allows another molecule to traverse a membrane which the molecule would not or not sufficiently fast be able to traverse without the transmembrane carrier or with the help of a membrane transport protein.

A "bioactive molecule" is understood as any molecule that has an effect when transported in a cell; this may include peptides, antibiotics, dyes and proteins.

A "membrane" may be understood as a sphere-shaped lipid layer that segregates an interior aqueous medium from an exterior aqueous solvent.

A "vesicle" may be understood as a synthetic unilamellar or multilamellar lipid bilayer structure that encapsulates an aqueous phase and that can be used as a cellular lipid bilayer model.

A "cluster compound", in particular a boron cluster compound, is understood as a molecule that is composed out of at least eight boron atoms and that has a molecular size below 2 nm in any dimension. Clusters (or "cluster compounds") are furthermore delimited from nanoparticles, as clusters are always soluble (in suitable media) and do not show a Tyndall effect. Furthermore, "multinuclear boron clusters", including "binuclear boron clusters", are known. Binuclear boron clusters, for example, consist of two of the above described (mononuclear) boron clusters, which are connected by a metal atom, for example cobalt, to form a such complex.

Boron cluster chemistry is dominated by icosahedrally shaped cages (see Assaf et al. ChemPhysChem 2020, 21, 97-976), which can be exemplified by closo-B₁₂H₁₂²⁻. Larger clusters ("fused clusters") can be formally obtained by their mutual fusion. The closo,closo-[B₂₁H₁₈]-ion is an example of shared icosahedral moieties with three joint vertices. The COSAN ion (CObalt SANdwich, Co(C₂B₉H₁₁)²⁻) represents another way of fusion, i.e., via a single vertex and an inner cobalt atom.

In one embodiment, the boron cluster compound is comprised in an aqueous solution which additionally comprises the membrane and the bioactive molecule, wherein the membrane is part of a cell or a vesicle so that a transport of the bioactive molecule inside of the cell or the vesicle occurs.

The boron clusters of the present invention can be used to transport a bioactive molecule from the aqueous solvent through the membrane into the cell or vesicle. Of course, it is also possible to transport the bioactive molecule from the cell or vesicle into the aqueous solvent using the boron cluster. The aqueous solvent is part of the aqueous solution, whereby the solution contains at least the aqueous solvent, the bioactive molecule, the boron cluster and the cell or vesicle.

According to one embodiment the boron cluster comprises a structure of the formula [BₐC_{b}X_{c}R_{d}Hₑ]^{p-}, wherein B represents boron, C represents carbon, X represents a halogen, R represents an organic group or a group comprising one or more heteroatoms and H represents hydrogen, wherein a is 8 to 22, b 0 to 4, c 0 to 26, d 0 to 26 and e 0 to 26, wherein c+d+e are 8 or greater but no greater than a+b and p is 1 to 4, wherein R may be the same or different groups, R may be optionally linked to the remainder of the molecule by a linker and X may be the same or different halogens.

According to another embodiment the boron cluster comprises a structure of the formula [BₐC_{b}X_{c}R_{d}Hₑ]^{p-}, wherein B represents boron, C represents carbon, X represents a halogen, R represents an organic group or a group comprising one or more heteroatoms and H represents hydrogen, wherein a is 8 to 15, b 0 to 2, c 0 to 17, d 0 to 17 and e 0 to 17, wherein c+d+e are 8 or greater but no greater than a+b and p is 1 to 4, wherein R may be the same or different groups, R may be optionally linked to the remainder of the molecule by a linker and X may be the same or different halogens.

In one embodiment, R is a C1 to C20 organic group with or without heteroatoms.

In another embodiment, R is a C1 to C20 organic group with heteroatoms.

In yet another embodiment, R is a C1 to C7 organic group with or without heteroatoms.

In one embodiment, R is a group with heteroatoms comprising 30 or less atoms overall.

A group comprising heteroatoms is understood as a group which comprises one or more heteroatoms and may or may not comprise carbon and/ or hydrogen, wherein the heteroatom is or the heteroatoms are preferably selected from nitrogen, oxygen, sulfur, phosphorus, fluorine, bromine, chlorine and iodine. The group comprising heteroatoms may in particular be a nitrobenzoxadiazole (NBD) group, a nitro group (-NO₂), a sulfonic acid group (-SO₃H), a trimethylsilyl group [-Si(CH₃)_{3]} a trifluoromethyl group (-CF₃), an amine group (-NH₂, -NHR, -NR₂ or -NR₃⁺) or a thiol group (-SH) .

In one embodiment, the boron cluster has more than one R group which can be the same or different.

The group R may be optionally connected with the boron atoms B or carbon atoms C of the boron cluster via a linker. In this case, the linker may, for example, be a thiomorpholine or an aminoalkyl group.

In one embodiment R comprises a nitrobenzoxadiazole (NBD) group.

In yet another embodiment, p is 1 or 2 so that the boron cluster has a single or double negative charge.

In one embodiment, the structure of the formula [BₐC_{b}X_{c}R_{d}Hₑ]^{p-} represents the entire boron cluster.

In one embodiment, the boron cluster is of globular or ellipsoidal shape.

Globular shape means in this context that the boron atoms are essentially arranged in a globular shape. Ellipsoidal shape means in this context that two globular boron cages are fused in a molecule to afford an ellipsoidally elongated structure. In particular, the term globular or ellipsoidal shape does not exclude pendant groups, such as an n-propoxy group as in the case of B2. Thus, B2 is also understood as a boron cluster of globular shape and CS3-CS6 are also understood as boron clusters of ellipsoidal shape.

In one embodiment, the boron cluster is a mononuclear boron cluster, a part of a multinuclear boron cluster or a part of a fused boron cluster.

In a preferred embodiment, the boron cluster is B₁₂Br₁₂²⁻ , B₁₂Br₁₁OC₃H₇ ²⁻, B₁₂H₁₁NBD⁻ or B₁₀B₁₀²⁻ or any of (1,2-C₂B₉H₁₁)₂-3,3'-Co⁻, (1,7-C₂B₉H₁₁)₂-2, 2'-Co⁻, (1,2-(CH₃)₂-1,7-C₂B₉H₉)₂-3,3'-Co⁻, (8-I-1,2-C₂B₉H₁₀)-(1'2-C₂B₉H₁₁)-3,3'-Co⁻, 8,8'-Cl₂-(1,2-C₂B₉H₁₀)₂-3,3'-Co⁻ or 8,8'-I₂-(1,2-C₂B₉H₁₀)₂-3,3'-Co⁻.

In a further embodiment, the boron cluster has an intrinsic affinity to hydrophobic binding sites and high biocompatibility.

Intrinsic affinity to hydrophobic binding sites can be probed, for example, through an affinity of macrocyclic host molecules such as cyclodextrins as described, for example, in El Anwar et al., Chem. Commun. 2019, 55, 13669-13672. Biocompatibility can be probed through a retained stability of lipid bilayer vesicles or through cellular toxicity assays.

In another embodiment, the bioactive molecule is cationic, zwitterionic or not charged.

In one embodiment, the boron cluster compound is a fused boron cluster or a multinuclear boron cluster.

In a further embodiment, the boron cluster compound is negatively charged.

### Detailed description of the invention

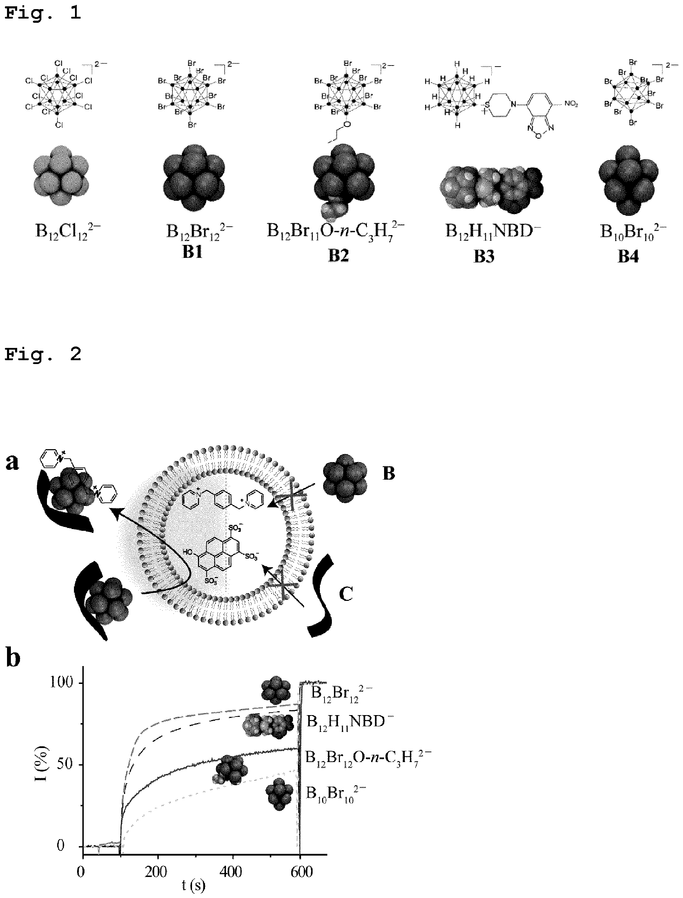

In the following, the invention is explained by providing examples. In the figures shows
- Figure 1: Chemical structures (top, ● = Boron) and space-filling molecular models (bottom) of dodecaborate (B₁₂X₁₂²⁻) and decaborate (B₁₀Br₁₀²⁻) clusters, and selected derivatives, whereas particularly effective boron clusters are marked with B1 to B4,
- Figure 2: a, schematic representation of the transport of otherwise impermeable analytes facilitated by superchaotropic anions with encapsulated HPTS/DPX probe/quencher pair employed for signaling (B: Boron Cluster, C: Impermeable cargo). b, Changes in HTPS emission (λₑₓ = 413 nm, λₑₘ = 511 nm) in EYPC⊃HPTS/DPX (13 µM phospholipids) as a function of time during the addition of carriers B1-4 (namely: B1: B₁₂Br₁₂²⁻, B2: B₁₂Br₁₁-O-n-C₃H₇²⁻_{,} B3: B₁₂H₁₁NBD⁻, and B4: B₁₀Br₁₀²⁻) ; clusters (150 µM) added at t = 50 s, heptaarginine (20 µM) at t = 100 s, and TX-100 at t = 600 s, for calibration; (I (%): Fluorescence intensity normalize to 100%, t(s): time in seconds),

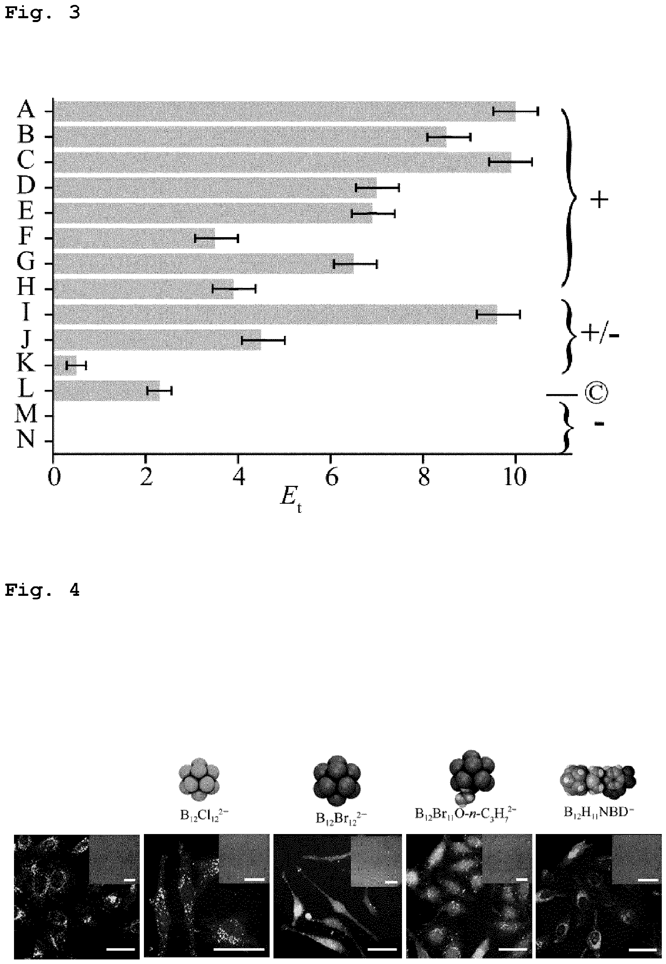

- Figure 3: the transport efficiency of B1 towards selected impermeable analytes of biological/clinical relevance; error bars refer to standard deviation. A: Heptaarginine, B: Heptalysine, C: Protamine, D: Acethylcholine, E: Pancuronium, F: Vecuronium, G: Vitamin B1, H: Ranitidin, I: Tryptophan, J: Ampicilin, K: Phenylalanine, L: Phalloidin, M: Glutamic acid, and N: Bovine albumin. +: positively charged cargos, +/-: zwitterionic cargos, ^{©}: neutral cargo, and -: negatively charged cargos,
- Figure 4: TAMRA-octaarginine (Tm-Args) endosomal escape promoted by different boron clusters (structures on top) in HeLa cells. Cells were incubated with 1 µM Tm-Arg8 and 0 (left picture) or 10 µM clusters diluted in HKR buffer for 1 hour, washed twice with HKR buffer, and imaged by confocal fluorescence microscopy; Images show TAMRA fluorescence (light gray) and the brightfield in the inset pictures; scale bars: 50 µm;

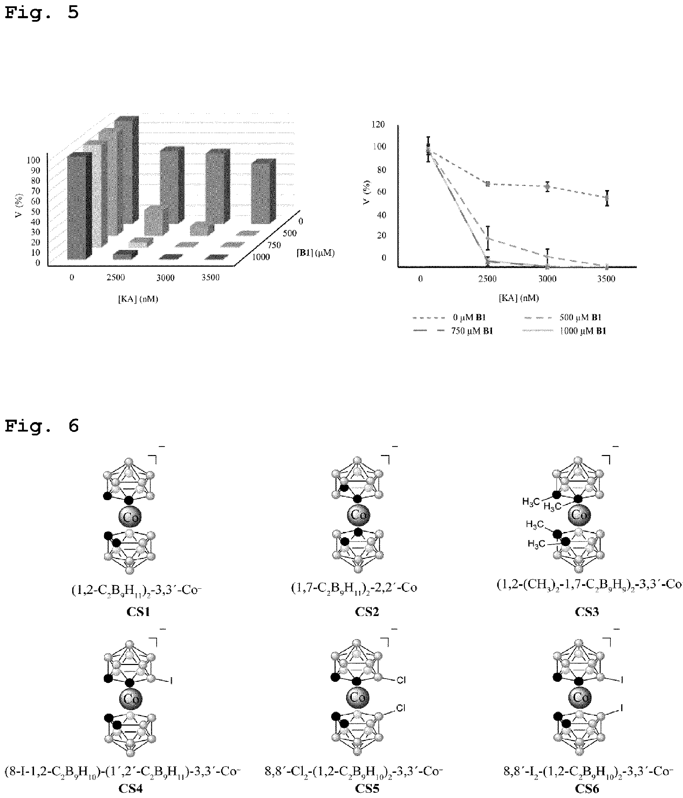

- Figure 5: B1-mediated kanamycin activity enhancement. Bar graphs (left) and time course (right) of *E. coli* Top10 viability in the presence of different concentrations of kanamycin monosulfate (0, 2500, 3000 or 3500 nM) and B1 (0, 500, 750 or 1000 µM) in LB broth at 37°C; error bars (shown on the right) refer to the standard deviation (n = 3; V (%): *E. coli* Top10 viability, [KA] (nM): Kanamycin A concentration in nanomolar, and
- Figure 6: chemical structures of COSAN cluster derivatives. (● = Boron, ● (grey) = Carbon).

### Examples

The synthesis of boron clusters and their chemistry is well developed due to their previous consideration for applications in boron neutron scattering therapy. Several boron clusters are commercially available. When boron clusters bear several or a single functional group, in particular OH, SH, or NH₂, they can be converted in many cases to derivatives, including organic derivates, by standard reaction procedures. Examples of such synthetic conversions of boron clusters under formation of organic derivatives are given in Assaf et al. Org. Lett. 2016, 18, 932-935, or El Anwar et al. Chem. Commun. 2019, 55, 13669.

A hydrophilic heptaarginine peptide (H-Trp-Arg₇-OH) which is unable to spontaneously translocate across neutral phosphocholine lipid vesicles in the absence of counterion activators was used as prototype for cationic cell-penetrating molecular scaffolds (peptides and polymers). The capability of boron clusters to activate the transport of the heptaarginine peptide (H-Trp-Arg₇-OH) was investigated first in large unilamellar vesicles (LUVs). The HPTS/DPX assay, that uses 8-hydroxypyrene-1,3,6-trisulfonate and *p-*xylene-bis-pyridinium, was implemented to monitor peptide-transport activation by the clusters. This assay not only reports on the potential of a synthetic carrier to transport the positively charged peptide into a vesicle, but also to shuttle the cationic quencher DPX to the outside.

Transport can accordingly be monitored, and the activator efficacy quantified, by a time-resolved fluorescence increase at different concentrations of the carrier.

HTPS emission was monitored during the sequential addition of the globular cluster carrier and peptide cargo; a strong detergent (Triton X-100) was added at the end to affect vesicle lysis and complete dye release that allowed a normalization of the fluorescence intensity data. All chlorinated and brominated clusters were positive hits: They did not cause membrane disruption but instead acted as efficient transporters of heptaarginine across the lipid membrane of the vesicles.

For the highly active brominated cluster, a single short alkoxyl group (B₁₂Br₁₁O-propyl²⁻) has little influence. On the other hand, when a 7-nitrobenzofurazan group is attached to the parent cluster (B₁₂H₁₁NBD-), efficient transport is observed.

In a further investigation it could be shown that boron clusters are suitable for the transport of neutral molecules, zwitterionic molecules and cationic molecules.

This was validated in vesicle transport experiments, which included targets which have not been previously accessible to other non-covalent synthetic carriers or counterion activators. The targets included singly charged, zwitterionic, and neutral biomolecules (such as acetylcholine and amino acids), vitamins, antibiotics, neuromuscular blocking agents and proteins. The cluster B₁₂Br₁₂²⁻ (B1) transported all types of cargo (positive and neutral), with the exception of the negatively charged molecules (glutamate and albumin).

Most striking was the very fast transport kinetics, within seconds, for most cargo types, except for the neuromuscular blocking agents and phenylalanine.

The same trends were reproduced in transport efficiency with the smaller brominated cluster, B4 (B₁₀Br₁₀²⁻) which proves that the cargo scope is transferable to different globular cluster carriers.

As the comparison, the same experiment was performed with an amphiphilic counterion activator from the state of the art, pyrene butyrate. None of the selected, zwitterionic or neutral, analytes (acetylcholine, tryptophan, ampicillin, vecuronium and phalloidin) showed any fluorescence response using the amphiphilic counterion activator of the state of the art. This demonstrates that the achieved transport phenomena and cargo scope are unique to borate clusters.

Such transport is also possible into the interior of living cells. For example, it was possible to transport the cell dye phalloidin, which is known in cell biology as an excellent cell dye for the cytoskeleton, but which can only be transported into living cells with great effort. It could furthermore be shown that in one case an antibiotic could also be transported in bacteria (kanamycin), which increased its effectiveness.

The potential of boron clusters to trigger the endosomal escape into live cells was first studied for oligocationic peptides as cargo. As a prototype, we selected the octaarginine peptide labelled with carboxytetramethylrhodamine (Tm-Arg₈), which at low concentrations (i.e. 1 µM) enters cells by endocytosis and remains trapped in the intracellular compartments. The cytosolic delivery of Tm-Args was followed by confocal microscopy in the presence and absence of superchaotropic boron clusters (Fig. 4) . In the absence of the clusters, cells incubated with Tm-Args showed punctate fluorescence of the penetrating peptide. In the presence of cluster B1-3, which had been identified as most active carriers in vesicles, we observed enhanced peptide endosomal escape as shown by the diffuse cytosolic fluorescence. Peptide delivery experiments with the fluorescent B3 cluster showed again significant peptide and cluster colocalization and heterogeneous intracellular distribution together with excellent peptide endosomal escape. The borate clusters showed furthermore very low cellular toxicity at different concentrations (as judged by MTT assay) also in the presence of 1 µM of the Tm-Args peptide.

Another area where carrier function is intensively being sought for is pharmaceutical drug delivery. As a proof-of-principle for pharmaceutical drug delivery the potential of the prototype chaotropic boron cluster carrier, B1, was studied in reducing the minimum inhibitory concentration (MIC) of kanamycin A, a polycationic aminoglycoside antibiotic.

For kanamycin A, effective passage through the cell membrane into the cytosol is essential to reach its intracellular targets. For example, the action of kanamycin A (3.5 µg/ml) on the Gram-negative Escherichia coli Top10 strain was investigated in the absence and presence of B1 (500 µM, compatible with mammalian cell survival). In the absence of the cluster, E. coli retained viability (60%), which disappeared almost completely (<5%) in the presence of the antibiotics carrier (Fig. 5). The enhanced antibiotic activity is attributed to the boron cluster's carrier potential as all other factors were left the same.

Also fused boron clusters and multinuclear boron clusters were successfully used as transporters for a bioactive molecule through a membrane.

Also iodinated boron clusters, in particular partially iodinated boron clusters, were successfully used as active clusters, and compounds CS4 or CS6 and the larger clusters such as CS1 or CS2 (COSAN clusters).

## Claims

1. Use of a boron cluster compound as transmembrane carrier to transport a bioactive molecule across a membrane of a cell or vesicle.

2. Use of a boron cluster according to claim 1, wherein the boron cluster comprises a structure of the formula [BₐC_{b}X_{c}R_{d}Hₑ]^{p-}, wherein B represents boron, C represents carbon, X represents a halogen, R represents an organic group or a group comprising one or more heteroatoms and H represents hydrogen, wherein a is 8 to 22, b 0 to 4, c 0 to 26, d 0 to 26 and e 0 to 26, wherein c+d+e are 8 or greater but no greater than a+b and p is 1 to 4, wherein R may be the same or different groups, R may be optionally linked to the remainder of the molecule by a linker and X may be the same or different halogens.

3. Use of a boron cluster according to claim 2, wherein R is an C1 to C20 organic group with or without heteroatoms.

4. Use of a boron cluster according to claim 2, wherein R comprises a nitrobenzoxadiazole (NBD) group.

5. Use of a boron cluster according to one of claims 2 to 4, wherein p is 1 or 2 so that the boron cluster has a single or double negative charge.

6. Use of a boron cluster according to one of claims 2 to 5, wherein the structure of the formula [BₐC_{b}X_{c}R_{d}Hₑ]^{p-} represents the entire boron cluster.

7. Use of a boron cluster according to one of the previous claims, wherein the boron cluster is of globular or ellipsoidal shape.

8. Use of a boron cluster according to one of claims 1 to 5, wherein the boron cluster is a mononuclear boron cluster, a part of a multinuclear boron cluster or a part of a fused boron cluster.

9. Use of a boron cluster according to one of claims 2 to 5, wherein the boron cluster is B₁₂Br₁₂²⁻, B₁₂Br₁₁OC₃H₇²⁻, B₁₂H₁₁NBD⁻ or B₁₀Br₁₀²⁻ or any of (1,2-C₂B₉H₁₁)₂₋3,3'-Co⁻, (1,7-C₂B₉H₁₁)₂-2,2'-Co⁻, (1,2-(CH₃)₂-1,7-C₂B₉H₉)₂-3,3'-Co⁻, (8-I-1,2-C₂B₉H₁₀)-(1'2'-C₂B₉H₁₁)-3,3'-Co⁻, 8,8'-Cl₂-(1,2-C₂B₉H₁₀)₂-3,3'-Co⁻ or 8,8'-I₂-(1,2-C₂B₉H₁₀)₂-3,3'-Co⁻.

10. Use of a boron cluster according to one of the previous claims, wherein the boron cluster has an intrinsic affinity to hydrophobic binding sites and high biocompatibility.

11. Use of a boron cluster according to one of the previous claims, wherein the bioactive molecule is cationic, zwitterionic or not charged.
